# EUROPEAN PATENT APPLICATION

(11) **EP 1 710 304 A1**
(43) Date of publication of application: **11.10.2006**
(21) Application number: 04807651.7
(22) Date of filing: 24.12.2004
(51) Int. Cl.: C12N 9/96, A61K 38/43, A61P 35/00, A61P 43/00, C12N 9/88

(54) **PROCESS FOR PRODUCING POLYMER COMPOSITE OF PROTEIN**

(30) Priority: 24.12.2003 JP 2003426601
(71) Applicant: Shionogi Co., Ltd., Osaka-shi, Osaka 541-0045 (JP)
(72) Inventor: TAKAKURA, Tomoaki, Shionogi & Co., Ltd., Amagasaki-shi, Hyogo 6600813 (JP); NOTSU, Yoshihide, Shionogi & Co., Ltd., Amagasaki-shi, Hyogo 6600813 (JP); TAKIMOTO, Akio, Shionogi & Co., Ltd., Amagasaki-shi, Hyogo 6600813 (JP)
(74) Representative: Vossius & Partner
(86) International application number: PCT/JP2004/019294
(87) International publication number: WO 2005/061701

(57) **Abstract**

By reacting a protein conjugated with a polymer thereto with a compound having a mercapto group and eliminating a polymer ester-bound to a mercapto group of a cysteine residue, a protein-polymer complex can be produced without considerably reducing function of a protein.

## Description

### Technical field

The present invention relates to a method for eliminating a polymer which is ester-bound to a mercapto group of a cysteine residue of a protein, and a process for producing a protein-polymer complex using the method. More particularly, the present invention relates to a process for producing a protein-polymer complex, comprising a step of reacting a protein conjugated with a polymer thereto with a compound having a mercapto group to eliminate a polymer which is ester-bound to a mercapto group of a cysteine residue of the protein.

### Background art

Generally, when a protein itself is used as a drug, there are problems that a blood half-life is short, antigenicity is high, storage stability is deteriorated, solubility is low, and so on.
In order to solve the above problems, it is known that a blood half-life can be prolonged, antigenicity can be reduced, and storage stability can be improved by conjugating a polymer such as polyethylene glycol etc. to a protein (Patent Literature 1). It is known that methioninase (L-methionine γ-lyase) can reduce an amount of methionine essential in proliferation of a tumor cell, can selectively suppress proliferation of a tumor cell without impairing healthy tissue, and has anti-tumor effect (Patent Literature 2). In addition, recombinant methioninase (Patent Literature 3), methioninase with polyethylene glycol bound thereto (Patent Literature 4), and methioninase, function of which has been altered by amino acid substitution, are also known.
Patent Literature 1: USP No. 4,179,337
Patent Literature 2: Japanese Patent No. 2930723
Patent Literature 3: Japanese Patent No. 3375834
Patent Literature 4: Japanese Patent Application National Publication (Laid-Open) No. 2002-531050

### Disclosure of the invention

### Problems to be solved by the invention

Generally, a protein conjugated with a polymer such as polyethylene glycol etc. thereto has the greatly reduced function as compared with a protein before conjugation, and is not suitable in utilization as a drug in some cases. Under such the circumstances, a process for producing a protein-polymer complex without greatly reducing function of a protein has been demanded.

### Means to solve the problems

The present inventors studied a process for producing a methioninase-polymer complex using methioninase as an example of a protein, and found out that (i) when polyethylene glycol is bound to methioninase, the activity of methioninase is greatly reduced, (ii) the reduction in activity is caused by an ester bond between a polymer and a mercapto group of a cysteine residue in methioninase, (iii) the ester bond can be selectively cleaved with dithiothreitol etc., (iv) activity of methioninase conjugated with polyethylene glycol after treatment with dithiothreitol etc. is about 1.5 to 4-fold higher activity before treatment with dithiothreitol etc., and (v) the activity is about 70 % or more of activity of original methioninase. Similarly, increase in activity was observed also in papain and transglutaminase.

Based on the aforementioned findings, the present inventors completed the following inventions.
(1) A process for producing a protein-polymer complex, comprising a step of reacting a protein conjugated with a polymer thereto with a compound having a mercapto group to eliminate a polymer which is ester-bound to a mercapto group of a cysteine residue of the protein.
(2) The process according to claim 1, wherein the protein conjugated with a polymer thereto is obtained by reacting a protein having a cysteine residue with an activated polymer.
(3) The process according to claim 1, wherein the polymer is polyalkylene oxide.
(4) The process according to claim 3, wherein the polymer is polyethylene glycol.
(5) The process according to claim 1, wherein the compound having a mercapto group is any of dithiothreitol, dithioerythritol, 2-mercaptoethanol, reduced glutathione and N-acetyl-L-cysteine.
(6) The process according to claim 1, wherein the compound having a mercapto group is dithiothreitol or 2-mercaptoethanol.
(7) The process according to claim 1, wherein the protein is an enzyme.
(8) The process according to claim 7, wherein the enzyme contains a cysteine residue in an active center.
(9) The process according to claim 8, wherein the enzyme is methioninase, papain or transglutaminase.
(10) The process according to claim 1, wherein average 0.7 to 1.3 molecules of a polymer are eliminated per 1 subunit of a protein.
(11) A protein-polymer complex obtained by a process as defined in any one of claims 1 to 10.
(12) The process according to claim 1, wherein the protein-polymer complex is a methioninase-polyethylene glycol complex, papain- polyethylene glycol complex or transglutaminase-polyethylene glycol complex.
(13) A methioninase-polyethylene glycol complex, papain-polyethylene glycol complex or transglutaminase-polyethylene glycol complex obtained by a process as defined in claim 12.
(14) A methioninase-polymer complex, which has average 3.1 or more of free mercapto groups per 1 subunit.
(15) An anti-tumor agent, containing a methioninase-polymer complex as defined in claim 13 or 14.
(16) A method for eliminating a polymer which has ester-bound to a mercapto group of a cysteine residue of the protein, comprising reacting a protein conjugated with a polymer thereto with a compound having a mercapto group.

### Effect of the invention

According to the process of the present invention, a polymer which has ester-bound to a mercapto group of a cysteine residue of a polymer conjugated with a polymer thereto can be eliminated. In addition, by using the present invention, a protein-polymer complex can be produced without greatly reducing function of a protein. A protein-polymer complex obtained by using the present invention not only has a long blood half-life, has reduced antigenicity, and is improved in storage stability, but also has high pharmacological activity, therefore, is useful as a drug.

### Brief description of the drawings

[Fig. 1] Fig. 1 is a view showing results of measurement of an absolute molecular weight at a subunit of DTT-untreated PEG-rMETase and DTT-treated PEG-rMETase using MADLI-TOF/MS.
[Fig. 2] Fig. 2 is a view showing a profile of a plasma methionine concentration with time after DTT-treated PEG-rMETase (○) or DTT-untreated PEG-rMETase (□) was injected into a mouse tail vein.
[Fig. 3] Fig. 3 is a view showing a profile of a plasma methionine concentration with time after pump containing a PLP solution was transplanted in a mouse subcutaneously, and DTT-treated PEG-rMETase (●) or DTT-untreated PEG-rMETase (¦) was administered into a mouse tail vein.

### Best mode for carrying out the invention

The present invention can be performed by reacting a protein conjugated with a polymer thereto with a compound having a mercapto group.
A protein used in the present invention means a protein having a mercapto group, for example, means a protein containing a cysteine residue in an amino acid sequence thereof. Thereupon, as a size of a protein, the number of amino acid residues of 10 or more is preferable, and a molecular weight of 1,000 or more is preferable. Examples of such the protein include methioninase, papain, chymopapain, cathepsin, transglutaminase, protease, keratin, hemoglobin, albumin, metallothionein and the like. A protein used in the present invention may be isolated and purified from a natural product, or may be made by recombinant technique.
In addition, as a protein, an enzyme is preferable, particularly, an enzyme containing a cysteine group in an active center is preferable. An active center is also called an active site, and refers to a site involved in specific binding arrangement of a substrate, and a site manifesting catalytic action in an enzyme protein. Examples of such the enzyme include methioninase, papain, chymopapain, cathepsin, transglutaminase and the like.

A protein conjugated with a polymer thereto used in the process of the present invention can be obtained, for example, by reacting a protein having a cysteine residue with an activated polymer.
An activated polymer is used for binding a polymer to an amino group or a mercapto group in a protein. As an activated polymer, a commercially available product may be used, or the polymer may be prepared by activating a commercially available polymer. For example, a polymer which has been derived into active ester, etc. can be used. When there is a carboxyl group in a polymer, the carboxyl group may be converted into an active ester. Alternatively, when there is no carboxyl group in a polymer, a linker having a carboxyl group may be bound to convert the carboxyl group into active ester. As a linker, a linker which is usually used may be used, a linker of preferably a carbon number of 1 to 20, further preferably a carbon number of about 2 to 5 is used.
For example, methoxy polyethylene glycol etc. can be obtained from Union Carbide Corporation. Activation of a polymer can be performed using N-hydroxysuccinimide (NHS) or the like. Examples include methoxy polyethylene glycol succinimidyl propionate (MSPA), methoxy polyethylene glycol succinimidyl glutarate (MEGC) etc. 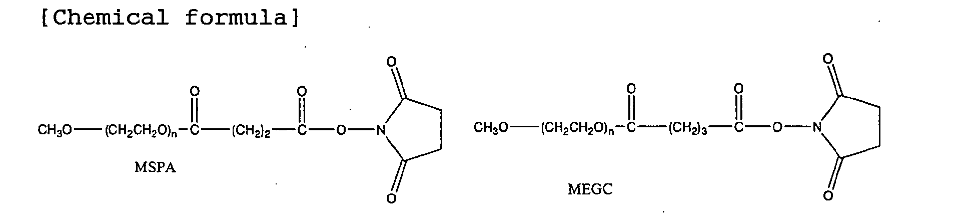 (wherein n is an arbitrary integer, preferably 50 to 1000) A polymer refers to a synthetically prepared high-molecular compound in which the same kind or some kinds of molecules (monomers) are bound into a chain, and it is particularly preferable that a polymer is water-soluble. Specifically, examples include dextran, poly(N-vinyl)pyrrolidone, polyalkylene oxide, polyoxyethylene polyol, polyolefin alcohol, polyacrylmorphan and the like and, particularly, polyalkylene oxide is preferable.
Examples of polyalkylene oxide include α-substituted polyalkylene oxide derivative, polyethylene glycol homopolymer, polypropylene glycol homopolymer, alkoxy polyethylene oxide, bis-polyethylene oxide, a copolymer of poly(alkylene oxide), and a block copolymer of poly(alkylene oxide) or an activated derivative. Particularly, alkoxy polyethylene oxide is preferable.
Examples of alkoxy polyalkylene oxide include alkoxy polyethylene glycol (e.g. methoxy polyethylene glycol etc.), alkoxy polyethylene oxide, and alkoxy polypropylene oxide. Particularly, methoxy polyethylene glycol is preferable.
As the polymer, a polymer having an average molecular weight of about 200 to about 50,000 daltons can be used. Preferable is a polymer having an average molecular weight of about 1,000 to about 20,000 daltons, further preferable is a polymer having an average molecular weight of about 2,000 to about 10,000 daltons, and most preferable is a polymer having an average molecular weight of about 4,000 to about 6,000 daltons.
A shape of the polymer may be straight, branched or comb-like. Since an amount of an activated polymer to be used is different depending on a kind and an amount of a protein to be used, it is necessary to appropriately adjust the amount, because the amount is influenced by the number of amino groups or mercapto groups in a protein, and so on. For example, when a protein is methioninase, the protein is a tetramer consisting of four subunits, and each subunit has ten amino groups (including an N-terminal amino group) and four mercapto groups. When 30 mole equivalents of an activated polymer is used on methioninase, around 4 to 6 polymers are bound per each subunit. In addition, when 60 mole equivalents of an activated polymer is used, around 7 to 10 polymers are bound per each subunit.
The protein-polymer complex refers to a protein-polymer complex obtained by reacting a protein conjugated a polymer thereto with a compound having a mercapto group to eliminate a polymer which has ester-bound to a mercapto group of a cysteine residue of the protein.
In addition, in the protein-polymer complex, it is not necessary that polymers which are ester-bound to mercapto groups of all cysteine residues present in the protein are eliminated, but complexes in which a part of the polymers are eliminated are also included. A complex in which at least average 0.7 to 1.3 molecules of a polymer are eliminated per one subunit is preferable.

In order to produce a protein-polymer complex by the process of the present invention, first, a polymer (e.g. alkyl polyethylene glycol) is bound to a protein. Then, a compound having a mercapto group is reacted with a protein conjugated with a polymer thereto to eliminate an ester-bound polymer from a mercapto group of a cysteine residue of a protein, thereby, an objective protein-polymer complex can be obtained.
Specifically, the complex can be prepared as follows: first, in order to bind a polymer to a protein, a polymer is reacted with a carboxylating agent to introduce a carboxyl group into the polymer. The polymer with a carboxyl group introduced therein is reacted with a carboxyl group-activating agent to prepare an active ester entity of a polymer. Finally, by reacting the active ester entity with a protein, a protein conjugated with a polymer thereto can be obtained. For example, Japanese Patent Application National Publication (Laid-Open) No. 62-501449, Japanese Patent Application Laid-Open (JP-A) No. 10-87815, and so on may be referenced.
The number of polymer molecules which are bound to a protein varies depending on the number of active groups, for example, reactive groups present on a protein molecule, for example, an amino group and a mercapto group. Alternatively, the number also varies depending on the condition when a protein is reacted with a polymer, such as a temperature, a pH, a protein concentration, and so on. A protein conjugated with a polymer thereto obtained herein has a merit that it has a longer half-life, and has little immune antigenicity as compared with a protein conjugated without polymer thereto, but activity thereof is generally decreased in many cases.
The thus obtained protein conjugated with a polymer thereto is reacted with a compound having a mercapto group to eliminate a polymer which is ester-bound to a mercapto group of a cysteine residue of the protein, thereby, a protein-polymer complex can be prepared.
The compound having a mercapto group may be a compound having a free mercapto group and, particularly, a low-molecular compound is preferable. Examples include dithiothreitol, dithioerythritol, reduced glutathione, N-acetyl-L-cysteine, 2-mercaptoethanol and the like and, particularly, dithiothreitol and 2-mercaptoethanol are preferable.
An amount of the compound having a mercapto group to be used is preferably around 0.01 to 10 % by weight relative to a weight of a reaction solution.

Methioninase is a degrading enzyme having a substrate of methionine, and is known to have anti-tumor activity (e. g. Kreis et al., Cancer Res., 33:1862-1865 and 1866-1869, 1973). Methioninase is a homotetramer consisting of four subunits, and a molecular weight of each subunit is about 43 kDa. An amino acid sequence thereof is represented, for example, by SEQ ID NO: 1, and ten amino groups (including a N-terminal amino group) and four mercapto groups are possessed in the sequence.
As an embodiment of the present invention, a process for producing a polymer complex by methoxy polyethylene glycol of methioninase will be exemplified below. First, a method of binding methoxy polyethylene glycol to methioninase will be explained. Methoxy polyethylene glycol having an average molecular weight of about 1,000 to about 20,000, preferably about 2,000 to about 10,000, most preferably about 4,000 to about 6,000 daltons is succinated or glutarated using succinic anhydride, preferably glutaric anhydride. Subsequently, using preferably N-hydroxysuccinimide, activated methoxy polyethylene glycol is obtained. Finally, activated methoxy polyethylene glycol is reacted with methioninase.

Then, a step of treating methioninase conjugated with methoxy polyethylene glycol thereto with a compound having a mercapto group will be explained. About 1 to 200 mole equivalents of a compound having a mercapto group is reacted per 1 molecule of methioninase with methoxy polyethylene glycol bound thereto at 4 to 50°C, preferably 10 to 40°C. Thereupon, as a reaction solvent, acetonitrile, dimethylformamide, dimethyl sulfoxide etc. can be used. A reaction time is 10 minutes to 4 hours, preferably 30 minutes to 2 hours.
In order to isolate and purify objective methioninase conjugated with methoxy polyethylene glycol thereto from the resulting reaction solution, a normal method of purifying a protein (e.g. Molecular Cloning 3rd edition, Cold Spring Harbor Laboratory, 2001) is used. That is, precipitation, membrane separation, column chromatography, high performance liquid chromatography, crystallization etc. are used.

Regarding methioninase, comparison was performed before and after conjugating with a polymer, and before and after treatment with a compound having a mercapto group.
First, the number of mercapto groups of methioninase before conjugating with a polymer was measured. Theoretically, there are four mercapto groups per 1 subunit, but an actually measured value was average about 3.7/subunit. Then, the number of mercapto groups of methioninase after binding with a polymer was measured. As a result, the number of mercapto groups was average about 2.7/subunit.
Then, the number of mercapto groups of methioninase after treatment with a compound having a mercapto group was measured. As a result, the number of mercapto groups was average about 3.7/subunit.
By the present method, average about 1 molecule (e.g. 0.7 to 1.3 molecules) of a polymer per one subunit of a protein can be eliminated. This was almost the same when an activated polymer to be reacted was used at 30 mole equivalents per methioninase, and when at 60 mole equivalents. From these results, it is seen that, when a polymer is bound, among four mercapto groups present per one subunit of methioninase, average one mercapto group was ester-bound to a polymer. Further, it is seen that when methioninase with a polymer bound thereto is treated with a compound having a mercapto group, a polymer ester-bound to the mercapto group is eliminated. It was also confirmed that, thereupon, a polymer amido-bound to an amino group of methioninase is not eliminated.
From these findings, it can be said that in a methioninase-polymer complex obtained by the process of the present invention, a polymer is not bound to a mercapto group. Actually, when a methioninase-polymer complex obtained by the present process is measured, for example, by an Ellman method (G. L. Ellman et al. , Arch. Biochem. Biophys., 82,70-77 (1959)), the complex has average 2.8 or more, preferably average 3.1 or more, further preferably average 3.5 or more free mercapto groups per one subunit.

Further, regarding methioninase, comparison was performed before and after conjugating with a polymer, and before and after treatment with a compound having a mercapto group. Details will be explained in Examples later, activity after treatment of methioninase conjugated with polyethylene glycol thereto with dithiothreitol or the like is about 1.5 to 4-fold higher than activity before treatment with dithiothreitol or the like. Increase in activity of an enzyme was also confirmed in a papain (SEQ ID NO: 2) polymer complex, and transglutaminase (SEQ ID NO: 3) polymer complex. That is, it can be said that the present process improves function of a protein as compared with a protein with a polymer bound thereto before reaction with a compound having a mercapto group. Herein, function of a protein means enzyme activity, receptor activity etc.

In addition, regarding methioninase, it has been suggested that a 116^{th} cysteine residue in an amino acid sequence thereof is important concerning a relationship with activity (Hiroyuki Tanaka et al., J. Biochemistry, 117, 1120-1125 (1995), Toru Nakayama, et al., Agric. Biol. Chem., 52, 177-183, (1988)). When the above result together with this report is taken into consideration, it can be said that there is a high possibility that a polymer ester-bound to 116^{th} cysteine of methionine was eliminated by treatment with a compound having a mercapto group. That is, there is a high possibility that a methioninase-polymer complex obtained by the process of the present invention is a composition comprising a methioninase-polymer complex in which 116^{th} amino acid is a free mercapto group.

A methioninase-polymer complex obtained by the process of the present invention can effectively inhibit proliferation of a tumor cell, and is useful for treating and/or preventing colon, breast, prostate, ovary, kidney, pharynx, melanoma, sarcoma, lung, brain, stomach, and bladder cancers. That is, an anti-tumor agent containing a methioninase-polymer complex obtained by using the present invention not only has low antigenicity but also retains high activity, and is useful as a drug.

When the protein-polymer complex obtained by the present invention is administered as a drug, it can be administered by any of oral and parenteral methods. For oral administration, the complex is prepared into normally used dosage forms such as tablets, granules, powders, capsules, pills, solutions, syrups, buccal preparations and sublingual preparations according to the conventional method, and they may be administered. For parenteral administration, the complex can be suitably administered in any normally used dosage form such as injectables for intramuscular administration, intravenous administration etc., suppositories, transdermally absorbing agents, inhalants etc. For example, a methioninase-polymer complex obtained by the process of the present invention can be used in intravenous drip infusion or the like.

If necessary, an effective amount of the protein-polymer complex obtained by the present invention can be mixed with various medical additives suitable for a dosage form such as excipients, binders, wetting agents, disintegrating agents, lubricants, diluents and the like to obtain pharmaceutical preparations. In the case of injectables, the complex together with an appropriate carrier may be sterilization-treated to obtain preparations.
Specifically, examples of excipients include lactose, white sugar, glucose, starch, calcium carbonate and crystalline cellulose, examples of binders include methylcellulose, carboxymethylcellulose, hydroxypropylcellulose, gelatin and polyvinylpyrrolidone, examples of disintegrating agents include carboxymethylcellulose, sodium carboxymethylcellulose, starch, sodium alginate, agar powder and sodium laurylsulfate, and examples of lubricants include talc, magnesium stearate and macrogol. As a base for suppositories, cacao butter, macrogol, methylcellulose etc. can be used. When the complex is prepared into solution preparations or emulsion or suspension injectables, solubilizing aids, suspending agents, emulsifiers, stabilizers, preservatives, isotonics and the like which are normally used may be appropriately added and, in the case of oral administration, corrigents, fragrances and the like may be added.
For example, the methioninase-polymer complex obtained by the process of the present invention may be used after lyophilization.

It is desirable to set a dose of the protein-polymer complex obtained by the present invention as a drug in view of an age, and a weight of a patient, a kind and a degree of a disease, an administration route and the like and, when orally administered to an adult, the dose is usually in a range of 0.05 to 200 mg/kg/day, preferably 0.1 to 100 mg/kg/day. In the case of parenteral administration, the dose is greatly different depending on an administration route, and is usually in a range of 0.005 to 20 mg/kg/day, preferably 0.01 to 10 mg/kg/day. This may be administered by injection or drip infusion by dividing into once to a few times per day. The dose is different depending on a kind of a protein. Therefore, a dose outside the aforementioned dose range may be used.

### Examples

### Example 1

The present invention will be explained below using recombinant methioninase (hereinafter, described as rMETase in some cases) as a protein. The present invention is not limited to Examples.
Process of producing dithiothreitol (DTT)-treated polyethylene glycol-conjugated recombinant methioninase (PEG-rMETase)
(i) rMETase This was obtained according to Japanese Patent Application No. 9-270676
(ii) Step of concentrating and preparing rMETase A 2-fold amount of a 120 mM sodium borate buffer (pH 9.0) was added to about 170 g of rMETase, followed by mixing. Then, this was concentrated to 100 g/L using a 1.8 m² membrane having a molecular weight cutoff of 10 kDa. 150 g of concentrated rMETase was used in a step of PEG conjugation.
(iii) Step of PEG conjugation and DTT treatment 275.4 g of activated PEG (MEGC-50HS, manufactured by NOF Corporation) was added to 150 g of rMETase in three portions (91.8 g×3) under a constant temperature of 37°C every 10 to 15 minutes. A pH was adjusted to 7.2 with a 100 mM disodium hydrogen phosphate solution after a reaction for 10 to 15 minutes from third addition. DTT was added to 0.5 % concentration, and the mixture was stirred under a constant temperature of 37°C for 2 hours to react materials, which was stored at 15°C overnight.
(iv) Diafiltration step A 50 mM sodium phosphate buffer, pH 7.2 was added so that a total amount of the resulting reaction solution became 36.0 L, followed by mixing. Diafiltration was performed using a 2.1 m² membrane having a molecular weight cutoff of 50 kDa while 42 L of a 50 mM sodium phosphate buffer, pH 7.2 was added at a flow rate of 0.7 L/min. Subsequently, this was concentrated to about 3 L. A protein in a membrane was recovered using 2 L of a 50 mM sodium phosphate buffer, pH 7.2, and mixed with a concentrated solution to obtain a Diafiltration-treated solution.
(v) Ion exchange column chromatography step The Diafiltration-treated solution was supplied at a flow rate of 50 cm/h to a DEAE Sepharose-FF column (φ140×65 mm, 1 L) equilibrated with a 50 mM sodium phosphate buffer, pH 7.2, to obtain a pass through fraction. A 50 mM sodium phosphate buffer, pH 7.2 was added to an eluant to adjust to 12.0 L, and this was filtered with a 0.2µm, 0.05 m² membrane, and stored at 15°C.
(vi) Gel filtration step An ion exchange column chromatography eluant was supplied at a flow rate of 11 cm/h to a Sephacryl S200 HR column (φ600×600 mm, 170 L) equilibrated with a 10 mM sodium phosphate buffer, pH 8.0. Active fractions were collected, and concentrated using a 30 kDa, 1.2 m² membrane. A protein in a membrane was recovered using about 1 L of a 10 mM sodium phosphate buffer, pH 8.0, and a 10 mM sodium phosphate buffer was further added to adjust a protein concentration to about 40 g/L.
(vii) Step of adding pyridoxal phosphate (PLP) A PLP solution adjusted to a 1 mM concentration was added to a concentrated solution at an amount which is 1/9 of the amount of the concentrated solution, this was mixed, and each about 200 mL was dispensed into a bottle having a volume of 250 mL using a sterilized 0.22 µm, 0. 02 m² membrane, and stored at -80°C.

### Example 2

### Increase in specific activity by DTT treatment (methioninase)

Specific activities of DTT-untreated PEG-rMETase and DTT-untreated rMETase, and DTT-treated PEG-rMETase and DTT-treated rMETase were measured. Activity measurement was performed according to Japanese Patent Application No. 9-270676. As a result, as shown in Table 1, by performing PEG conjugation, considerable decrease in specific activity was observed, but by performing DTT treatment, the activity was greatly recovered.

**[Table 1]**

| (1) Effect of DTT treatment on specific activity of PEG-rMETase (reaction mole ratio 60) | | |
|---|---|---|
| | DTT treatment | Specific activity (U/mg) |
| rMETase | - | 56 |
| | + | 56 |
| PEG-rMETase | - | 16 |
| | + | 44 |

Then, the same experiment was performed at a reaction mole ratio of 30 using activated PEG (manufactured by NOF Corporation) having a different molecular weight from that of MEGC-50HS. As a result, as shown in Table 2, by performing DTT treatment, recovery of specific activity was observed even in activated PEG having a different molecular weight.

**[Table 2]**

| Effect of DTT treatment on specific activity of PEG-rMETase prepared using activated PEG having a different molecular weight | | | |
|---|---|---|---|
| | Molecular weight of activated PEG | DTT treatment | Specific activity (U/mg) |
| MEGC-50HS | 5 kDa | - | 33 |
| | | + | 45 |
| MEGC-10TS | 10 kDa | - | 32 |
| | | + | 47 |
| MEGC-20TS | 20 kDa | - | 33 |
| | | + | 47 |

Further, when the same experiment was performed at a reaction mole ratio of 30 using other PEG modifier (MSPA5000, manufactured by NEKTAR) having a different linker from that of MEGC-50HS, and other PEG modifier (MENP-50H, manufactured by NOF Corporation) having a different active group from that of MEGC-50HS, as shown in Table 3, recovery of specific activity was observed even in other PEG modifiers by performing DTT.

**[Table 3]**

| Effect of DTT treatment on specific activity of PEG-rMETase prepared using activated PEG having different linker and active site | | | | |
|---|---|---|---|---|
| | Linker | Active group | DTT treatment | Specific activity (U/mg) |
| MEGC-50HS | Glutaryl | NHS | - | 33 |
| | | | + | 45 |
| MSPA5000 | Propionyl | NHS | - | 33 |
| | | | + | 38 |
| MENP-50H | Carbonyl | Paranitrophenol | - | 10 |
| | | | + | 30 |

Effect of DTT treatment in reaction between protein other than methioninase and MEGC-50HS
(1) Using papain (manufactured by Wako Pure Chemical Industries, Ltd.), a PEG conjugation reaction and DTT treatment were similarly performed, and specific activities thereof were compared. For assessing activity of papain, a reaction was performed in a 50 mM Tris hydrochloride buffer (pH 7.5) containing 0.1 mM L-cysteine, 2 mM EDTA · 2 Na, and 1 mM N-α-benzoyl-DL-arginine-p-nitroanilide hydrochloride, and an amount of p-nitroaniline produced at 37°C for 1 minute was monitored and measured at 410 nm. As a result, as shown in Table 4, it was found out that specific activity is considerably decreased by PEG conjugation, but the activity is recovered by performing DTT treatment.

**[Table 4]**

| Effect of DTT treatment on specific activity of PEG conjugated papain | |
|---|---|
| | Relative specific activity (%) |
| Papain | 100 |
| Reaction mixture of PEG conjugation | 42 |
| DTT treatment | 106 |

(2) Actinomyces, Streptomyces sp. was cultivated with shaking at 30°C for 3 days using a medium (pH 7.0) consisting of 2.0 % polypeptone, 2.0 % soluble starch, 0.2 % dipotassium hydrogen phosphate, 0.1 % magnesium sulfate, and 0.1 % yeast extract. Bacterial cells were removed by centrifugation, this was dialyzed with a 25 mM sodium borate buffer (pH 8.5) to obtain a crude enzyme solution of transglutaminase (TGase). Four mg of MEGC-50HS was added to 0.25 mL of this crude enzyme solution to react materials at 25°C for 1 hour. DTT treatment was performed at 0.1 % of a final concentration of the reaction solution, and specific activity was compared. TGase activity was measured according to the method of Folk J. E. and Chung S. I. (Methods Enzymol. 113, 358-375, (1985)). As a result, as shown in Table 5, specific activity of TGase was reduced to 36 % by PEG conjugation, but recovered to 70 % by performing DTT treatment.

**[Table 5]**

| Effect of DTT treatment on specific activity of PEG conjugated transglutaminase | |
|---|---|
| | Relative specific activity (%) |
| TGase | 100 |
| Reaction mixture of PEG conjugation | 36 |
| DTT treatment | 70 |

From the forgoing, it can be said that the effect due to DTT treatment is not limited to rMETase, but can be also applied to other enzymes.

### Example 3

DTT has a SH group and a hydroxy group as shown below. 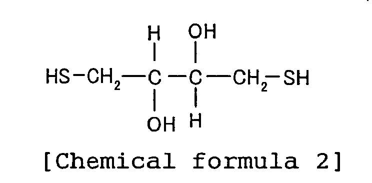 Whether increase in specific activity by DTT treatment is caused by a SH group or a hydroxy group was investigated. A compound having a SH group (dithiothreitol, reduced glutathione, N-acetyl-L-cysteine, 2-mercaptoethanol), or an alcohol having a hydroxy group (methanol, ethanol, methoxyethanol, 1-propanol, 2-propanol, 1-butanol, 2-butanol, 2-methyl-1-propanol) was reacted with PEG-rMETase obtained by the previous method, and specific activity was compared. As a result, as shown in Table 6, recovery of specific activity was confirmed only when contacted with a compound having a SH group, and it was suggested that increase in specific activity due to DTT treatment is caused by a SH group.

**[Table 6]**

| Compounds influencing on recovery of specific activity of PEG-rMETase | |
|---|---|
| Compound | Relative specific activity (%) |
| *N*-Acetyl L-cysteine | 79 |
| Glutathione (reduced) | 55 |
| 2-Mercaptoethanol | 70 |
| Dithiothreitol | 100 |
| Methanol | 5 |
| Ethanol | 4 |
| Methoxyethanol | 4 |
| 1-Propanol | 4 |
| 2-Propanol | 4 |
| 1-Butanol | 5 |
| 2-Butanol | 1 |
| 2-Methyl-1-propanol | 5 |
| No addition | 5 |

| | |
|---|---|
| An enzyme solution was prepared using a buffer without DTT. | |

### Example 4

### Quantitation of remaining SH group

rMETase is a recombinant protein of methioninase, and has four SH groups per one subunit. An amount of a liberated SH group present in rMETase accompanied with a PEGylating reaction and DTT treatment was quantitated using an Ellman method after a protein had been denatured by sodium dodecylsulfate (SDS) treatment.
(1) Change in SH group amount in PEG-rMETase production process As shown in Table 7, rMETase before reaction with activated PEG (MEGC-50HS) was 3.7 SH/subunit, but by a PEGylating reaction, it became 2.7 SH/subunit, and decrease of 1 SH/subunit was observed. Then, when treated with 0.5 %DTT, a remaining SH group of PEG-rMETase was recovered to 3.7 SH/subunit, becoming an equivalent value to that of rMETase.
(2) Further, the reaction mixture of PEG conjugation obtained by the same method was divided into two, and a DTT-treated entity and an untreated entity were prepared, a remaining SH group was quantitated and, as a result, as shown in Table 8, a DTT-untreated PEG-rMETase became 3.0 SH/subunit, and DTT-treated PEG-rMETase became 3.7 SH/subunit, showing an approximately equivalent value to that in change in the production step.
(3) From these results, it can be thought that, in a PEGylation step, average one SH group per one subunit is reacted with activated PEG (MEGC-50HS), and PEG is liberated by DTT treatment. In addition, it is presumed that a SH group from which PEG has been liberated is a site greatly influencing on activity.

**[Table 7]**

| The number of SH group in step of producing DTT-treated PEG-rMETase | |
|---|---|
| Step | SH/subunit |
| rMETase | 3.7 |
| Reaction mixture of PEG conjugation (reaction mole ratio 60) | 2.7 |
| DTT-treated PEG-rMETase | 3.7 |

**[Table 8]**

| Remaining SH group of DTT-untreated/treated PEG-rMETase | |
|---|---|
| PEG-rMETase (reaction mole ratio 60) DTT treatment | SH/subunit |
| - | 3.0 |
| + | 3.7 |

### Example 5

### Modification of cysteine residue (Cys) using monoiodoacetic acid (MIA)

After rMETase, DTT-treated PEG-rMETase which had been DTT-treated after PEGylation of rMETase as described above, or DTT-untreated PEG-rMETase was reacted with MIT known as a SH modifier, this was further DTT-treated. The results are shown in Table 9. By modifying a SH group with MIA, specific activity was considerably decreased in all of DTT-untreated PEG-rMETase and DTT-treated PEG-rMETase. Thereafter, as a result of reaction by adding DTT, increase in specific activity was observed only in DTT-untreated PEG-rMETase. This is thought as follows : since DTT-untreated PEG-rMETase had a SH group which had been conjugated with PEG in advance, it could not undergo SH group modification with MIA and, by performing DTT treatment, PEG was eliminated from a SH group, and activity of an enzyme was increased.

**[Table 9]**

| Influence of DTT treatment and MIA modification on specific activities of rMETase, DTT-untreated PEG-rMETase, and DTT-treated PEG-rMETase | | | |
|---|---|---|---|
| | Specific activity (U/mg) | | |
| | rMETase | DTT-untreated PEG-rMETase | DTT-treated PEG-rMETase |
| Before MIA reaction | 54 | 17 | 38 |
| After MIA reaction | 2.4 | 3.8 | 4.0 |
| DTT treatment | 2.7 | 12 | 4.6 |

From results of Examples 4 and 5, the following is found. That is, although by reacting rMETase with activated PEG, in specific activity is considerable decreased, by DTT treatment, the activity was greatly recovered. Since there is a report that, among four Cys residues per one subunit of rMETase, Cys at 116^{th} from a N-terminus is an important factor involved in expressing enzyme activity (Hiroyuki Tanaka et al., J. Biochemistry, 117, 1120-1125 (1995), Toru Nakayama, et al., Agric. Brol. Chem., 52,177-183, (1988)), it was presumed that PEG selectively conjugated Cys116 involved in expressing the enzyme activity, and DTT eliminates only the PEG moiety from Cys116. A possibility of PEG conjugation to Cys was studied and, as a result, increase in specific activity was observed also in a SH group-containing compound other than DTT. The number of SH groups of rMETase was 3.7 SH/subunit, and was reduced to 2. 7 SH/subunit by a PEGylating reaction. Thereafter, by performing DTT treatment, this was recovered to 3.7 SH/subunit. In addition, by modifying a SH group with MIA, considerable decrease in specific activity was observed, but by performing DTT treatment, specific activity was increased only in DTT-untreated PEG-rMETase. From the forgoing results, it can be concluded that activated PEG (MEGC-50HS) modifies a SH group (Cys) playing an important role in expressing enzyme activity of rMETase.

### Example 6

### Quantitation of the number of bound PEGs of PEG-rMETase (MALDI-TOF/MS)

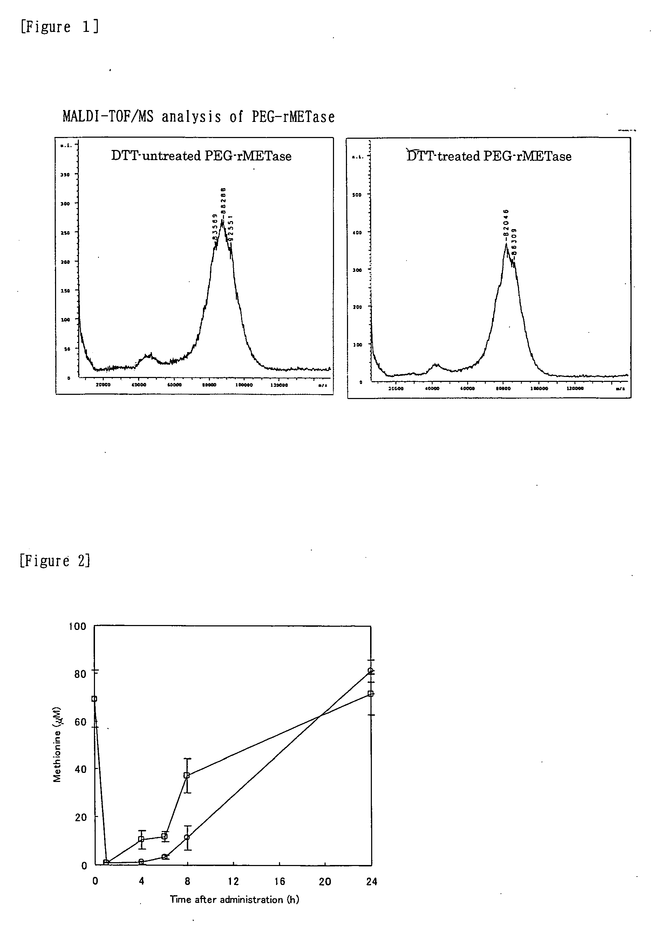

An absolute molecular weight of DTT-untreated PEG-rMETase and DTT-treated PEG-rMETase at a subunit level was analyzed by MALDI-TOF/MS. As a result, as shown in Fig. 1, it was found out that a molecular weight of DTT-treated PEG-rMETase was decreased by about 5 kDa relative to DTT-untreated PEG-rMETase. Since a molecular weight of PEG is about 5 kDa, it was thought that 1 PEG/subunit was eliminated by DTT treatment.

### Example 7

### Quantitation of the number of bound PEGs of PEG-rMETase (HPLC)

The number of conjugated PEGs of DTT-untreated PEG-rMETase, and DTT-treated PEG-rMETase was measured (John Bullock et al. , Aanl. Biochem., 254,254-262, (1997)). PEG which had been liberated by alkali hydrolysis was measured with HPLC by a quantitation method using PEG4120 as a standard. As a result, as shown in Table 10, in the case of DTT-untreated PEG-rMETase, the number was 8.4 PEG/subunit, while in the case of DTT-treated PEG-rMETase, the number was 7.5 PEG/subunit, and decrease of about 1 PEG/subunit was observed. In addition, the similar result was obtained also at a reaction mole ratio of 30, and the number of bound PEGs was decreased by about 1 PEG/subunit by DTT treatment.

**[Table 10]**

| The number of conjugated PEGs in PEG-rMETase | | |
|---|---|---|
| PEG-rMETase DTT treatment | Reaction mole ratio 60 PEG/subunit | Reaction mole ratio 30 PEG/subunit |
| - | 8.4 | 5.0 |
| + | 7.5 | 4.1 |

### Example 8

### Measurement of degree of modification on amino group by fluorescamine

A degree of modification on amino group of DTT-untreated PEG-rMETase and DTT-treated PEG-rMETase was measured using rMETase as a control by the Fluorescamine method (Laurel J. Karr et al., Methods in Enzymology, 228, 377-390 (1994)). As a result, as shown in Table 11, the degree of modification on amino group was almost equal irrespective of the presence or the absence of DTT treatment. Therefore, it was confirmed that PEG bound to an amino group does not cause elimination accompanied with DTT treatment.

**[Table 11]**

| Degree of modification on amino group in PEG-rMETase | |
|---|---|
| PEG-rMETase (reaction mole ratio 60) DTT treatment | Degree of modification (%) |
| - | 75 |
| + | 74 |

As a result of DTT treatment, increase of about 1 SH group per PEG-rMETase subunit, decrease in an absolute molecular weight of about 1 PEG molecule, and elimination of about 1 PEG molecule were observed. However, a degree of modification on amino group was unchanged. From them, it can be concluded that PEG conjugated to a SH group is easily eliminated by DTT treatment.

From the forgoing, it is thought that PEG forms an unstable thioester bond with Cys playing an important role in expressing methioninase activity to decrease specific activity in a step of PEG conjugation reaction, but an exchanging reaction occurs by performing DTT treatment, and PEG-rMETase having a recovered SH group is produced.

### Example 9

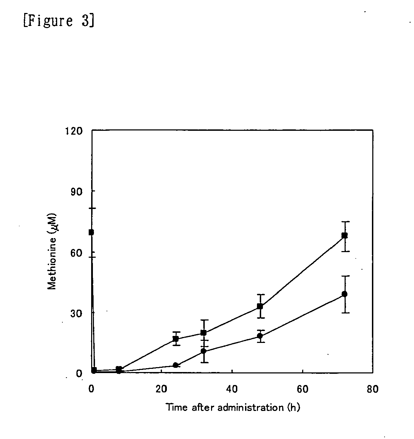

The same protein amount (140 mg/kg) of DTT-treated PEG-rMETase or DTT-untreated PEG-rMETase which had been prepared at a reaction mole ratio of 60 was injected into a mouse tail vein (each group 3 animals). A blood was collected with time, and a plasma methionine concentration was measured (Barry N. Jones and James P. Gilligan, J. Chromatogr. 266,471-482 (1983)). The results are shown in Fig. 2. DTT-treated PEG-rMETase (o) maintained a plasma methionine concentration at a low level for a long time as compared with DTT-untreated PEG-rMETase (□), and effect of DTT treatment was confirmed in vivo. Further, the results when the same experiment was performed by transplanting a pump containing a PLP solution into a mouse subcutaneously are shown in Fig. 3. DTT-treated PEG-rMETase (●) could maintain a plasma methionine concentration at a low level for a long time as compared with DTT-untreated PEG-rMETase (¦). It was presumed that it is possible to decrease a necessary dose for treatment by performing DTT treatment.

### Example 10

Antigenicity was compared between DTT-treated PEG-rMETase and rMETase which had been prepared at a reaction mole ratio of 60. DTT-treated PEG-rMETase or rMETase was intraperitoneally administered to a mouse at a dose of 0.1 mg/animal once a week at a total of 12 times and, two weeks after final administration, a blood was collected to obtain a serum (each group 10 animals). Antigenicity was measured using an enzyme immunological measuring method. rMETase was fixed on a well of a polystyrene plate, and this was blocked and washed. The diluted serum was added to a well, this was washed, and a mouse anti-IgM antibody or anti-IgG antibody labeled with peroxidase was added. Serum of a mouse to which rMETase or PEG-rMETase had not been administered was used as a control, and a titer was measured. The number of individuals at each serum dilution rate is shown in Table 12. Antigenicity of DTT-treated PEG-rMETase was considerably decreased than rMETase.

**[Table 12]**

| Antibody class | Antigen | Dilution rate (Log) | | | | | | | |
|---|---|---|---|---|---|---|---|---|---|
| | | 0 | 1 | 2 | 3 | 4 | 5 | 6 | 7 |
| Ig M | rMETase | 0 | 0 | 3 | 6 | 1 | 0 | - | - |
| | DTT-treated PEG-rMETase | 8 | 0 | 1 | 1 | 0 | 0 | - | - |
| Ig G | rMETase | 0 | 0 | 0 | 0 | 4 | 4 | 2 | 0 |
| | DTT-treated PEG-rMETase | 0 | 0 | 6 | 1 | 1 | 2 | 0 | 0 |

## Claims

1. A process for producing a protein-polymer complex, comprising a step of reacting a protein conjugated with a polymer thereto with a compound having a mercapto group to eliminate a polymer which is ester-bound to a mercapto group of a cysteine residue of the protein.

2. The process according to claim 1, wherein the protein conjugated with a polymer thereto is obtained by reacting a protein having a cysteine residue with an activated polymer.

3. The process according to claim 1, wherein the polymer is polyalkylene oxide.

4. The process according to claim 3, wherein the polymer is polyethylene glycol.

5. The process according to claim 1, wherein the compound having a mercapto group is any of dithiothreitol, dithioerythritol, 2-mercaptoethanol, reduced glutathione and N-acetyl-L-cysteine.

6. The process according to claim 1, wherein the compound having a mercapto group is dithiothreitol or 2-mercaptoethanol.

7. The process according to claim 1, wherein the protein is an enzyme.

8. The process according to claim 7 , wherein the enzyme contains a cysteine residue in an active center.

9. The process according to claim 8, wherein the enzyme is methioninase, papain or transglutaminase.

10. The process according to claim 1, wherein average 0.7 to 1.3 molecules of a polymer are eliminated per 1 subunit of a protein.

11. A protein-polymer complex obtained by a process as defined in any one of claims 1 to 10.

12. The process according to claim 1, wherein the protein-polymer complex is a methioninase-polyethylene glycol complex, papain- polyethylene glycol complex or transglutaminase-polyethylene glycol complex.

13. A methioninase-polyethylene glycol complex, papain-polyethylene glycol complex or transglutaminase-polyethylene glycol complex obtained by a process as defined in claim 12.

14. A methioninase-polymer complex, which has average 3.1 or more of free mercapto groups per 1 subunit.

15. An anti-tumor agent, containing a methioninase-polymer complex as defined in claim 13 or 14.

16. A method for eliminating a polymer which has ester-bound to a mercapto group of a cysteine residue of the protein, comprising reacting a protein conjugated with a polymer thereto with a compound having a mercapto group.
